Europäisches Patentamt

European Patent Office

Office européen des brevets

(19)

(11) Publication number: **0 408 483 B1**

## (12) EUROPEAN PATENT SPECIFICATION

(45) Date of publication of patent specification: **25.01.95**    (51) Int. Cl.[6]: **A61M 5/172**

(21) Application number: **90500063.4**

(22) Date of filing: **03.07.90**

The file contains technical information submitted after the application was filed and not included in this specification

(54) **Drug delivery system.**

(30) Priority: **04.07.89 ES 8902365**

(43) Date of publication of application:
**16.01.91 Bulletin 91/03**

(45) Publication of the grant of the patent:
**25.01.95 Bulletin 95/04**

(84) Designated Contracting States:
**AT BE CH DE DK FR GB GR IT LI LU NL SE**

(56) References cited:
**EP-A- 0 164 904
DE-A- 2 741 344
US-A- 4 392 849
US-A- 4 525 775
US-A- 4 551 133**

**ADVANCES IN INSTRUMENTATION, vol. 39, parts 1,2, PROCEEDINGS OF THE ISA INTERNATIONAL CONFERENCE AND EXHIBIT, Houston, Texas, 22nd - 25th October 1984, pages 1082-1093, ISA, Research Triangle Park, NC, US; MOORE et al.: "Expert systems applications in industry"**

(73) Proprietor: **R.G.B. MEDICAL DEVICES S.A.
Basilica, 20
E-28020 Madrid (ES)**

(72) Inventor: **Borches Jacassa, Daniel
Caleruega, 31
E-28033 Madrid (ES)**
Inventor: **Ruiz Fernandez, Ricardo
Eduardo Dato, 3
E-28010 Madrid (ES)**

(74) Representative: **Gonzalez Vacas, Eleuterio
Calle Sagasta, 4
E-28004 Madrid (ES)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Description**

OBJECT OF THE INVENTION

As set out in the heading of this descriptive report, the present invention refers to an improved method for delivering vasoactive drugs using infusion equipment with a computerized device which controls and adjusts dosage automatically in a closed loop and is used to control blood pressure. This improvement is based on including a unit which automatically controls and adapts the dosage for different vasoactive medicine infusion pumps.

HISTORY OF THE INVENTION

Parenteral use of vasoactive medicines (nitrites and nitrates) is now common practice for the clinical treatment of blood pressure and other cardiovascular pathologies (miocardial infarction, angina, serious congestive heart failure, heart surgery, etc.). They are given intraveneously, by continuous infusion, in most cases. For some medicines the dosage is of the dosage-response kind and the infusion pattern is usually different for each patient. Even for the same patient this pattern varies with time. In these cases, the "manual" dosage is usually given by starting off with a very low dose which is gradually increased until the desired response is obtained. Once the objective has been achieved, continuous supervision is required to maintain the same response levels as well as constant modifications of the dosage by the staff in charge of this control.

In the case of fast acting drugs whose effect wears off quickly, special characteristics are involved with regard to dosage as well as to control dosage response. This process requires continuous monitoring with close supervision. This "manual" control of dosage and response, is very time consuming and therefore involves diverting attention from ether equally important tasks.

Different strategies have been considered traditionally in order to develop a safe and effective method of controlling dosage and response automatically, see for example the patient controlled infusion system disclosed in US-A-4 392 849, which is reflected in the preamble of claim 1. However, equipment which is sufficiently satisfactory for clinical purposes, does not exist as yet.

Ideally, a vasoactive drug infusion controller unit for blood pressure control, should achieve sustained hypotension, within a set time while maintaining it within close limits and, should react quickly and correctly when faced with any circumstances which attempt to upset the balance achieved.

Traditionally, algorithms for automatic control of physiological variables have been designed based on mathematical formulas which establish a direct and unique relationship between input variables (present and past data concerning blood pressure) and the output variable (dosage to be infused).

One of the most widely used control algorithms is the so called PID (Proportional, Integrator, Derivative) controller, by means of which the amount of drug infused is a preestablished linear combination of the error between present and desired output, its integral and derivative. In spite of the fact that this kind of control is easy to implement, in practice it has not been found satisfactory due to the fact that there is no preestablished method of selecting the relative weights of the three linear combination terms, when the description of the physiological system is unknown.

Another type of control is based on what are known as optimum algorithms, perhaps the most representative example of which is the so called "generalized minimum variance" one.

Optimum controllers assume that the system is not known and they calculate the control required from two aspects: 1) the system's estimated characteristics (for which a pattern is made beforehand); and 2) operating criteria which should be minimized (error, amount of drum infused, etc.).

The advantage of using this type of controller resides in the fact that very sophisticated calculation techniques exist; their benefits can be predicted and are "optimum" in the sense that they optimize a certain criterion.

As a disadvantage we can point out the fact that while they are capable of operating best when the system is represented precisely, control can be inadequate when the system is represented imprecisely. To represent the system precisely, the optimum controller requires permanent stimulation to obtain the characteristic parameters. Paradoxically, when the desired conditions are achieved, stimulation must be at a minimum (to maintain these) and therefore both the source of information and control is consequently lost.

Other controllers which have mostly failed to provide suitable benefits are those which tend to cancel the physiological system's poles. This is not feasible in practice, since an infinite number of poles are involved in characterizing the physiological system completely, and they vary with time based on multiple factors, the majority of which are random.

Taking into account also that physiological systems are also not well characterized (different sensitivity of patients to the same medicine, changes in the same patient's sensitivity to any drug in time (for example, tachyphylaxis, etc.), it is desirable to use controllers which either do not depend on the cardiovascular system to be controlled or, if they do so, adapt automatically to its changes.

The present invention provides an apparatus according to the definitions given in the independent claim 1.

The benefit of the controller proposed by the invention, which is based on improving methods of deliverying vasoactive drugs, is based on implementing a new control concept based on qualitative characterization of the variables to be controlled. This controller introduces logical analysis to describe those "very complex" or "badly defined" systems which do not accept precise mathematical analysis. Its main features are as follows: 1) it uses qualitative (linguistic) characterization of the control phases rather than quantitative variables (numerical ones); and 2) it establishes the relationships between variables by means of conditional statements.

This system "based on rules" is a means of treating with "imprecision", modelling the process of logical human thinking, in order to control systems which cannot be modelled exactly. The main difference between this approach to the problem and conventional techniques is that the system "based on rules" uses qualitative information, while the latter require rigid analytical relationships to describe the process.

This system attempts to simulate the process of human thinking, so it starts from the premise that human beings "categorize" information (including numerical values), with linguistic labels. For example, the blood pressure descent gradient can be classified as: "very quick", "quick", "acceptable", "slow", "very slow", etc.

The limits of these labels are not precisely defined numerically, but permit overlapping in such a way that, in accordance with other conditioning factors, any numerical value can belong to different labels.

The rules of Bool's algebra allow labels or groups of labels to be combined in order to establish the present condition from among the controller's range of possibilities and to take the relevant steps, based on this.

There are no standard rules for calibrating a control system based on rules such as the one described and, both prior experience as well as lots of experiments are required in order to identify the parameters used by the operator, deduce the linguistic labels used by the operator to classify the values measured for each parameter, and if possible, identify the range of each label and decide on the conditional rules which relate these linguistic labels to special actions of control.

This kind of control criterion is implemented by computerized equipment which by means of a closed loop system, compiles the analogical or digital information originating from a patient's monitoring (from conventional monitoring equipment, or from a transductor) and which, once the information has been processed, determined and sends dosage orders to an infusion pump.

The equipment has three basic units: a controller, a pressure monitor and an infusion pump with the possibility of combining these three devices in several ways: controller (connected to a conventional monitor and to an infusion pump of the kind sold at present), controller plus pressure monitor (connected to an infusion pump of the kind sold at present) or a controller plus a pressure monitor plus an infusion pump in the same unit.

In any case the infusion pump must be a high precision one and the pressure monitor must have a signal amplifying module with isolation for the blood pressure signal.

The main part of the equipment is the controller which includes a 256 K ROM memory, 64 K RAM microcontroller, visualizer which can generate its own characters, three standard RS-232 series communication outputs, real time clock, keyboard, indicators, microprocessor system back-up logic, battery, battery charging system, battery level detector and plate input level generator.

Among the indicators are included the on/off, battery level, running control and infusion order indicators. With regard to the microprocessor system's backup logic we can point out decodification, reset generator, pressure reference, latch, etc. The power supply board includes a DC-DC convertor, transformer with insulation, rectifier, regulator and mains-battery switch.

DESCRIPTION OF THE DRAWINGS

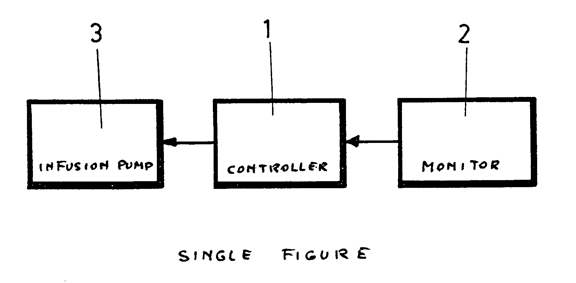

To complete the description being made and to help the characteristics of the invention to be better understood, a single sheet of drawings is attached to the present descriptive report, as an integral part of same, showing in a single figure, the configuration which permits improvements to methods for delivering vasoactive drugs by infusion pumps by means of a computerized device which automatically controls and adjusts dosage in order to control blood pressure. This is the most widely used configuration, from which the specific configurations already described in this descriptive report are derived.

BEST WAY OF MAKING THE INVENTION

A look at the drawing shows us that the improvement to methods for delivering vasoactive drugs claimed, is based on using a controller (1), which has a monitor (2) and an infusion pump (3). The three items can comprise a single unit or optionally, a commercial monitor (2) or monitor (2) and infusion pump (3) can be used, since the special characteristics of the invention are in the controller (1) to be precise.

As we have already said, the controller (1) is organized on the basis of a ROM AND RAM memory, microcontroller, a visualizer which is capable of generating its own characters, three standard series RS-232 communication outputs, a real time clock, keyboard, on/off indicators, as well as battery level, running control and infusion order indicators, as well as the microprocessor system backup logic. The microcontroller on its part will be organized based on a central processing unit, a series communication device, two parallel input/output communication doors of eight bits each, and eight analogical/ digital conversion channels. With regard to the microprocessor system backup logic, besides the decodifying devices, there is a reset generator, a pressure reference, a latch, etc.

It is also obvious that the controller will be assisted by a supply battery, with its corresponding charging system, a level detector and a power supply board. A DC-DC convertor will be included in the latter, as well as a transformer with insulation, the corresponding rectification and regulation circuits, as well as a mains-battery switch.

With regard to the monitor (2), it must include a signal amplification module with isolation for intraarterial pressure signal.

Finally, the infusion pump (3) must be a high precision one.

As a means of further explanation of the EQUIPMENT above described, and in order to provide a better understanding of the subject of the present patent, the hardware modules are indicated as well as unique or most significant operating features of the equipment.(As explained further abstracted in the claims 1,2). any longer in order for any expert on the matter to understand the implications of the invention and the advantages derived from same.

The materials, form, size and arrangement of the items may be varied provided that this does not involve altering the essential part of the invention.

The terms in which this report has been written should always be taken in the widest possible sense and are not restrictive.

**HARDWARE DESCRIPTION OF EOUIPMENT**

The equipment contains the following operating functional modules:
1) Microprocessor system.
2) Analog pressure signal adapter.
3) Switching Power supply.
4) Battery charger plus battery backup system.
5) Graphic Digital Display.
6) keyboard

Next, the most important electronic component are presented, for each different functional module:

**MODULE 1: MICROPROCESSOR SYSTEM**

Circuit components of module 1 are listed:
a) Microcontroler 68HC11, Motorola;
b) Three standard RS232c serial communication ;
c) Up to kbytes of RAM memory ;
d) Up to 256 kbytes of EPROM memory ;
e) 32 digital TTL compatible input-output;
f) 8 Analog/Digital converter;
g) Acoustic signal adapter;
h) Display Backlight Actuator ;
i) Alarm visual indicator;
j) keyboard controller;

**MODULE 2: ANALOG PRESSURE SIGNAL ADAPTER**

Circuit components of module 2 are listed. The input signal comes from the arterial blood pressure transducer.

a) Diferential Amplifier with isolation;
b) Computer programable amplifier;
c) Low-pass filter, with 1khz BandWidth;
d) Clamp and Overvoltage protection to 5 V maximum;

**MODULE 3: SWITCHING POWER SUPPLY**

Circuit components of module 3 are listed:
a) Fuse and Transformer;
b) Diode Bridge and regulator ;
c) Switching Power Supply +5v, 1A ;
d) Switching Power Supply +15v, 200mA;
e) Switching Power Supply -15v, 200mA;

**MODULE 4: BATTERY CHARGER PLUS BATTERY BACkUP SYSTEM**

Circuit components of module 4 are listed:
a) Battery charger ;
b) Fuse ;
c) Batteries ;

**MODULE 5: GRAPHIC DIGITAL DISPLAY**

The Graphic Digital Display is a component that integrates screen memory and controller, as well as all necessary elements in order to interconnect with module 1, by means of a TTL compatible digital signal with input-output ports. The comunication protocol is indicated in program instructions herein contained. There are many displays available that can be used. As an example, the VIkAY graphic series is proposed.

**MODULE 6: kEYBOARD**

Up to 16 key keyboard can be used, with either numerical and function keys. The keyboard is placed in the font pannel, together with 2 visual indicators, one for Power ON/OFF state, the second as an alarm indicator.

| EOUIPMENT CONTROLS | | |
|---|---|---|
| **ITEM** | **NAME** | **FUNCTION** |
| 1 | POWER Cord Connector | AC power cord connector with retaining mechanism. |
| 2 | DATA | For connection to external INTERFACE devices, such as DINAMAP or FINAPRES, or to an external computer. |
| 3 | PUMP CONNECTOR | Screw-type connectors for connection of pump (for example NEW PERFUSOR SECURA of B.Braun). |
| 4 | POWER ON-OFF | Controls the AC power to the monitor, plus the battery supply. |
| 5 | PRESSURE TRANSDUCER CONNECTOR | Screw-type connector for conexion of pressure transducer (for example, COMBITRANS, of B.BRAUN) |

## MOST SIGNIFICANT OPERATING FEATURES OF THE EOUIPMENT

- GENERAL FEATURES OF THE EQUIPMENT

  The EQUIPMENT (arterial blood pressure monitor and controller) has been designed as a small portable bedside system.

  The EQUIPMENT has been designed to provide operator convenience and reliability when monitoring blood pressure, and when acting as blood pressure supervisor, sending computer commands to the infusion pump in order to set the rate of drug to be delivered.

- DIFERENT SITUATIONS WHERE THE EQUIPMENT CAN BE USED

  As blood pressure monitor, the equipment finds the medical applications already widely documented in the specialized bibliography.

  As pressure supervisor and controller, this EQUIPMENT is indicated in Operating Room and Medical Intensive Care Unit treatment, in the cases where a strict control of arterial pressure level is mandatory. Hipertensive crisis, acute myocardial infarction, congestive cardiac failure, cardiovascular pre, per and post-operatory cardiac surgery, eclampsia, medical care (nephrology) are some examples of Intensive Care Unit applications. Controlled hypotension in neural surgery, otorrinolaryngology and plastic surgery (oscillometric non-invasive pressure measurement system) are some applications of Operating Room situations.

  Under manual control, the EQUIPMENT permits the infusion of any drug, so that its use is recomended whenever it is advisable the infusion of a predefined drug, and the registration in memory of data such as blood pressure and dosage infused, on a minute by minute basis.

- MICROPROCESSOR BASED DESIGN. System fully upgradable.

- BACk-UP BATTERY

  The pressure supervisor is battery-backed and can be used as portable monitor during patient transportation.

- CONNECTION TO INFUSION PUMP

  It can be connected via one cable to the NEW INFUSOR SECURA pump. The EQUIPMENT will feed the pump, (whenever there is a DC 3-20 volts input power connector available in the PUMP), and send computer based infusion commands in order to set the pump's infusion values. The requirements that pumps must fulfill are that they have a standard RS232-c or IEEE EIA485 (ELECTRONICS INDUSTRY ASOCIATION) computer interface connector. Due to the large available number of pumps of this kind in the medical field, it is imposible to refer to all of them. As an example, it can be used the B.Braun Infusor NEW PERFUSOR SECURA.

- MEAN ARTERIAL PRESSURE MEASUREMENT SOURCES

  While in monitor or control mode, pressure values can be taken from three different sources:

  1) Intraarterial measurement by means of a percutaneous catheter. 2) Non-invasive oscilometric system (for example, DINAMAP of Critikon) and 3) Non-invasive pletismographic system (for example, FINAPRES of Omeda).

- The EQUIPMENT AS PRESSURE MONITOR

  The EQUIPMENT can act as a monitor of blood pressure, installing the pressure transducer with a percutaneous intravenous catheter, the EQUIPMENT itself is actually designed with its own blood pressure monitor, making it unecessary the use of an external monitor. It contains all the necessary elements in order to adapt the pressure transducer input signal. There is a large number of available transducers. The only requisite they must fullfil is that they be excited with DC supply voltage ranging between 2 to 15 volts.As an example, itis indicated the B.BRAUN COMBITRANS pressure transducer.

- CONNECTION TO AN EXTERNAL BLOOD PRESSURE MONITOR.

  It can be also connected, besides its own monitoring system, to any external arterial blood pressure monitor (available in the medical field) containing an analog output signal in the range 0-5volts.

  It can be also connected to different non-invasive blood pressure monitors, such as Oscilometric and Pletysmographic based systems, provided they have a standard RS232-c or IEEE EIA485 (ELECTRONICS INDUSTRY ASOCIATION) computer interface connector. As an example, it can be use DINAMAP (from Critikon) and FINAPRES (Omeda).

- INTERFACE RS232-c OR IEEE EIA485 (ELECTRONICS INDUSTRY ASOCIATION)

  They are two Industry standard interface for compatibility with most external programable devices and computer systems. This connector is used for connection to predefined non-invasive pressure monitor and computers such as for example IBM compatible computers.

- ARTIFACT REJECTION.

  The pressure supervisor is capable of eliminating most noise and motion artifact (monitor and control modes).

- AUDIBLE/VISUAL ALARM SYSTEM
  Provides a visual and audible indication if cardiovascular measurements fall outside of operator-programmable high/low limits and of abnormal system conditions or hardware failure.
- GRAPHIC DIGITAL DISPLAY
  Easy to read. Any such DISPLAY can be used, provided it accepts comunication with microprocessor board via input-output digital pots. Power supply can be in the range of 3 to 15 volts. As an example, VIkAY graphic display is indicated.
- UNITS OF MEASUREMENT
  pressure is given in mmHg for pressure, and pulse in beats per minute (bpm).

**MONITOR MODE:**

- GRAPHIC DISPLAY
  Monitors arterial pressure by means of a percutaneous implanted catheter. Results are graphically displayed on easy-to-read graphical display.
- PARAMETERS CALCULATION
  Systolic and Diastolic are searched by a tracking algorithm. Mean Pressure and Pulse computation are performed.

**CONTROL MODE:**

- MANUAL AND AUTO CONTROL MODE
  Two different control modes can be presented:
  1) Manual: Any kind of drug can be used.
  2) Auto: with Sodium-Nitroprusside only.
- SURVEILLANCE MODE
  It can work in surveillance mode, starting infusion only when MAP is higher than a predefined value.
- PRESSURE CLAMP DETECTOR
  It works as a pressure clamp detector. When clamp conditions are encountered, an acoustic alarm is activated.
- PUMP PROTOCOL
  While in control mode, the EQUIPMENT will keep a continual communication protocol with the pump. Any failure to keep the connection will be detected within seconds.
- DATA PRESENTATION
  While in control mode, results of the last 30 minutes of each control are displayed on a graphic screen. Mean, Maximum and Medium Pressure are presented.
- DATA REGISTRATION
  While in control mode, results of dose and pressure values are registered on a minute by minute basis. This values can be later reviewed both on the screen, or sent to a computer for further analysis. The EQUIPMENT allows for 24 hour data registration.
- MANUAL MODE CONTROL
  In manual control mode dosage is manually determined from the Baricontrol unit. Any drug can be used. Dosage will be mantained in this value until a new infusion command is given.
- AUTO MODE CONTROL
  In auto control mode, only Sodium NItroprusside must be used. Dosage are updated every 20 seconds according with a Fuzzy - Rule based algorithm for the computation of optimal dosage.

**AUTOMATIC DOSAGE DETERMINATION**

- INTELLIGENT COMPUTATION
  The pressure supervisor carries out a sophisticated scheme for optimal dosage computation.
- ALGORITHM PRESENTATION
  The algorithm introduces a fuzzy logic and rule-based procedure as a means of capturing human expertize and dealing with uncertainty. The algorithm incorporates "Fuzzy" logic elements, combined with an analytical optimal search methodology, for optimal dose computation.
- PRESSURE TARGET
  The ultimate objective of the algorithm is to bring pressure values to the target pressure gap. Target gap oscilates between target pressure and 10 mmHg above.

- EASY TO UNDERSTAND

  At all times, Dosage updating is very reasonable as a consequence of the computation method.

- SMOOTH CONTROL

  Optimal Dose search is carried out continually. There is however, minimal over-excitation to the patient.

- MINIMAL DOSE INFUSION

  In order to achieve a predefined target, the EQUIPMENT will adapt permanently, in order to reach it with minimum overall dosage infused to the patient. The method results minimally aggressive.

- COMPUTATION SCHEME OF OPTIMAL DOSE

  The algorithm introduces a fuzzy logic and rule-based procedure as a means of capturing human expertize and dealing with uncertainty. The algorithm incorporates "Fuzzy" logic elements, combined with an analytical optimal search methodology, for optimal dose computation.

- CONTROL MECHANISM

  Every 20 seconds, a new dose infusion must be determined. For this purpose, the MAP value is obtained, from over 1000 measures taken during a 5 seconds period.

- MEAN ARTERIAL PRESSURE CATEGORIZATION

  Definition of a Target Pressure Value is done at the start of the control. The inmediate consequence is that Pressure values become categorized. From a given value of Blood Pressure, four distinct areas are developed:

  "Above Target Gap" Above Target Gap upwards.

  "Target Gap", 5 mmHg wide above target pressure. Whenever Pressure values remain in this gap, control is considered to have no error.

  "Below Target Gap", about 5 mmHg wide.

  "Risk Gap", 5mmHg below Pressure Target down wards. In this case, Dose infusion is always 0.

- OPTIMAL DOSE CONCEPT

  The ultimate objective of the algorithm is to bring pressure values to the target gap. Once the target gap is reached, the Optimal Dose is infused, in order to mantain pressure values within its bounds. The Optimal Dose value is a theoretical concept, by which we mean a dose that, continually infused, will mantain pressure values within the target gap. Of course, this value adaptively changes with time and with varying patient conditions, and a permanent updating is carried out, whenever the pressure evolution undergoes an excursion either above or below the target gap.

- DOSE DETERMINATION

  Two different steps are conducted in order to determine the new infusion value.

- FIRST STEP IN DOSE DETERMINATION

  First step is conducted following the fuzzy logic methodology. The objective is to determine what kind of action should be taken. This decision has only three possible options: to mantain, to increase or to decrement dose infusion values. The information required to perform this step is based on qualitative rather than cuantitative values of variables. Linguistic labels are associated to the values obtained of input variables such as last MAP sampled, trajectory followed by pressure (shape factor, mean slope value, etc) and pressure gap as well as proximity to target gap.

- DATA CATEGORIZATION

  These labels are then related through conditional declarations in order to categorize the information. Priority decisions will determine the kind of action to be taken whenever the labels yield a vague or undefined situation. Once this first step is carried out, it remains still the need to determine the quantitative value of new dose to be infused.

- SECOND STEP IN DOSE DETERMINATION

  The second step implements an optimal dose search algorithm based on a Rule-Based methodology. The objective is twofold: On one side, The new dose value must be determined. If MAP is not in the target gap, then new Infusion values are incremented or decremented relative to the actual dose value, through a non linear relationship with actual MAP. The Proportionality coeficient increases exponentially with MAP. On the other side, an updating of optimal dose value is performed. If MAP belongs to the target gap, then the new infusion value equals the optimal dose. Otherwise, optimal dose is increased or decreased more slowly, following a logarithmic incrementation coeficient. This logarithmic relationship adapts with the patient's varying situation by means of a growth factor.

```
/*

 *      PROGRAM INSTRUCTIONS.



 */
unsigned char punto[1664];  /* 16*105 puntos al monitorizar */
unsigned short dosis_sim[90]={
                    356,356,356,356,356,356,356,356,382,412,
                    440,468,710,466,0,0,0,0,0,148,
                    256,256,256,256,128,128,0,0,104,184,
                    184,184,184,184,184,92,92,92,82,84,
                    148,148,148,148,148,148,74,74,74,74,
                    116,116,116,116,116,116,116,116,116,58,
                    58,58,104,104,104,104,104,104,104,104,
                    104,104,104,104,104,104,104,104,104,104,
                    104,104,104,104,104,104,104,104,104,104
                    };


unsigned char presion_sim[90]={
                    89,89,90,89,90,90,90,90,91,91,
                    91,91,89,77,63,68,65,65,63,65,
                    74,76,74,71,69,66,64,64,67,71,
                    74,75,74,71,70,68,65,65,67,68,
                    70,72,72,72,71,70,69,68,67,70,
                    71,72,74,73,72,72,71,70,71,69,
                    69,70,71,73,73,73,73,72,72,72,
                    71,72,72,70,71,70,70,71,71,71,
                    71,71,71,71,71,71,71,71,71,71
                    };
```

```
unsigned char grafico_sim[110]={
                              20,20,19,19,18,18,18,18,17,17,
                              17,17,16,16,15,15,15,15,14,14,
                              13,13,18,18,21,21,25,25,27,27,
                              30,30,28,28,25,25,23,23,21,21,
                              20,20,19,19,18,18,18,18,17,17,
                              17,17,16,16,15,15,15,15,14,14,
                              13,13,18,18,21,21,25,25,27,27,
                              30,30,28,28,25,25,23,23,21,21,
                              20,20,19,19,18,18,18,18,17,17,
                              17,17,16,16,15,15,15,15,14,14,
                              13,13,18,18,21,21,25,25,27,27,
                              };


unsigned char fin_programa;
#include "stdio.h"
/*
 *    Declaracion de prototipos.
 */
void control_automatico(void);
void control_manual(void);
void monitorizar_presion(void);
void finalizar_programa(void);
void opciones_varias(void);
```

```
/*
 *    Funciones.
 */
void dibuja_presion_dosis(coord_x,presion,ant_dosis,dosis)
unsigned char coord_x;
unsigned char presion;
unsigned short ant_dosis;
unsigned short dosis;
{
  unsigned char j,p=0,a_d=0,d=0;
  unsigned short j2;
  if(coord_x == 1) ant_dosis = dosis;
  j=presion;while(j>=5){j-=4;p++;}
  j2=ant_dosis;while(j2>=21){j2-=20;a_d++;}
  j2=dosis;while(j2>=21){j2-=20;d++;}
  for (j=0;j<=p;j++)plot(71-j,coord_x+23);
  if(d>p){
      plot(71-d,coord_x+23);
      if (a_d<=d)
         {
         if (a_d >= p)
            for (j=(a_d+1);j<d;j++) plot(71-j,coord_x+23);
         else
            {
            for (j=(a_d+1);j<p;j++) unplot(71-j,coord_x+23);
            for (j=(p+1);j<d;j++) plot(71-j,coord_x+23);
            }
         }
```

```
        else
          for (j=(d+1);j<a_d;j++) plot(71-j,coord_x+23);
          }
      else {
        unplot(71-d,coord_x+23);
        if (a_d <= d)
          for (j=(a_d+1);j<d;j++) unplot(71-j,coord_x+23);
        else
            {
          if (a_d<=p)
            for (j=(d+1);j<a_d;j++) unplot(71-j,coord_x+23);
          else
              {
            for (j=(d+1);j<p;j++) unplot(71-j,coord_x+23);
            for (j=(p+1);j<a_d;j++) plot(71-j,coord_x+23);
              }
            }
          }
    }
void control_de_presion( modo )
char modo;  /* modo=0 : control automatico */
            /* modo=1 : control manual     */
  {
    extern struct param valor_inicial;
    extern char hay_alarma;
    extern char l_pressed,kb_nextin;
    extern int t_20s;
```

```c
unsigned short dosis[90];
unsigned char fin_control=0,fin_bloque=0,i=1,j,k,descenso_lento=0,
                 forma,simulado=0,
                 presion_final=70,presion_media,
                 presion_maxima=0,presion_minima=0xff, a_d,
                 presion_alarma=200 ,presion[90],
                 consulta,i_cont=0;
unsigned short total,dosis_ant=0,dosis_auxiliar,
                 dosis_inicial=50 /* 0.5ml/h */;
unsigned long p_sum;
short dosis_actual;
char fallo;


dosis_actual=dosis_inicial;
forma=modo;
while (!fin_control){
   kb_nextin=0;
   fin_bloque=0;
   hay_alarma = 0;
   while(!fin_bloque && !hay_alarma){
      consulta = 0;
      borra_grafico();
      if (modo) {
                 menu_4_v("CONTRL","MENU","Real","Simul");
                 display_string_h(7,2,"Control:");
                 if (!simulado) display_string_h(7,10,"REAL    ");
                       else    display_string_h(7,10,"SIMULADO");
                 }
          else  {
```

```
            menu_4_v("CONTRL","MENU","D.rap","D.len");
            display_string_h(7,2,"Descenso :");
            if (descenso_lento) display_string_h(7,12,"LENTO ");
                  else           display_string_h(7,12,"RAPIDO");
            }


      display_string_h(1,2,"0 Dosis I:");
      display_var(1,16,dosis_actual);
      display_string_h(3,2,"1 P.Final:");
      display_char(3,15,presion_final);
      display_string_h(5,2,"2 P.Alarm:");
      display_char(5,15,presion_alarma);


      kb_pressed=0;
      while(!kb_pressed);
      switch (kb_pressed){
            case 'A':  fin_bloque=1;
                       consulta = 1;
                       borra_grafico();
                       menu_4_v("DOSIS","MENU","","");
                       display_string_v(0,19,"          ");
                       display_string_v(0,18,"          ");
                       dibuja_ejes();
                       for (k=1;k<=i;k++){
        dibuja_presion_dosis(k,presion[k],dosis[k-1],dosis[k]);
                          }
                       inicializa_conversion();
                       break; /* Opcion sup. izda. */
```

```
case 'B':    fin_bloque=1;
             fin_control=1;
             break; /* Opcion inf. izda. */
case 'C':    if (modo) simulado=0;
                    else descenso_lento=0;
             break; /* Opcion sup. dcha. */
case 'D':    if (modo) simulado=1;
                    else descenso_lento=1;
             break; /* Opcion inf. dcha. */
case '0':    borra_grafico();
             display_string_h(3,5,"Introduzca");
             display_string_h(4,9,"la");
             display_string_h(5,4,"DOSIS INICIAL");
             display_string_h(6,6,"( ml./h. )");
             dosis_inicial=input_var(8,10,dosis_inicial);
             dosis_actual=dosis_inicial;
             break; /* Opcion 0 */
case '1':    borra_grafico();
             display_string_h(3,5,"Introduzca");
             display_string_h(4,9,"la");
             display_string_h(5,4,"PRESION FINAL");
             display_string_h(6,6,"( mm.Hg. )");
             presion_final=input_char(8,10,presion_final);
             break; /* Opcion 1 */
case '2':    borra_grafico();
             display_string_h(3,5,"Introduzca");
             display_string_h(4,9,"la");
             display_string_h(5,2,"PRESION ALARMA");
             display_string_h(6,6,"( mm.Hg. )");
```

15

```
                    presion_alarma=input_char(8,10,presion_alarma)

                    break; /* Opcion 1 */

          default :  break;

          }

     }

if (consulta == 1)

   {


   datos_perfusor(&fallo);

   alarmas_perfusor(&fallo);


   valor_inicial.presion_objetivo = presion_final;

   dosis_auxiliar = dosis_actual/10;

   valor_inicial.infusion_inicial = dosis_auxiliar;

   if (descenso_lento)    valor_inicial.ritmo_caida = 10;

        else        valor_inicial.ritmo_caida = 15;



   p_sum=0;total=0;

   t_20s=155;

   while (t_20s){

      inicializa_adctl(0,0,0,0,0,0);

      while( !fin_conversion() );

      a_d=*adrl_addr;

      p_sum=p_sum+a_d;

      total++;

      }
```

```
presion_media = 0;
    while (p_sum>total){p_sum=p_sum-total;presion_media++; }
    valor_inicial.presion_inicial = presion_media;
    inicia_control(valor_inicial);
    }
fin_bloque=0;
l_pressed=0;
while( (!fin_bloque) && (!fin_control) && (!hay_alarma)){
    t_20s=400;
    while (t_20s);
    p_sum=0;total=0;
    l_pressed=0;
    datos_perfusor(&fallo);
    if (hay_alarma) break;
    alarmas_perfusor(&fallo);
    if (hay_alarma) break;
    t_20s=155;
    while (t_20s){
        inicializa_adctl(0,0,0,0,0,0);
        while( !fin_conversion() );
        a_d=*adr1_addr;

        if(!total) presion_minima = presion_maxima = a_d;
        if(a_d>presion_maxima) presion_maxima=a_d;
        if(a_d<presion_minima) presion_minima=a_d;
```

```
p_sum=p_sum+a_d;
total++;
if (l_pressed=='A'){
    forma=forma+2;
    l_pressed=0;
    display_string_h (1,13,"        ");
    display_string_h (2,13,"        ");
    display_string_h (3,13,"        ");
    display_string_h (4,13,"        ");
    display_string_h (5,13,"        ");
    display_string_h (6,13,"        ");
    dosis_actual=input_var(9,18,dosis_inicial);
    if (!modo)
        {
        dosis_auxiliar = dosis_actual/10;
        valor_inicial.infusion_inicial = dosis_auxiliar;
        inicia_control(valor_inicial);
        }
    t_20s=0;
    }
}
presion_media=0;
while (p_sum>total){p_sum=p_sum-total;presion_media++; }
if (l_pressed == 'B') break;
if (simulado) presion_media=presion_sim[i];
presion[i]=presion_media;
if (!forma){
    dosis_actual = dosis_control(presion[i]);
    dosis[i] = dosis_actual;
```

```
        }
    else {
        dosis[i] = dosis_actual;
        }
if (simulado)  { dosis[i]=dosis_sim[i];dosis_actual = dosis[i]; }


if (forma>1) forma=forma-2;
if (dosis[i] < 0.1) dosis[i] = 0;
dibuja_presion_dosis(i,presion[i],dosis[i-1],dosis[i]);

if((dosis_actual == 0) && (dosis_ant == 0))   i_cont++;
else    i_cont = 0;
if (i_cont == 4)
    {
    i_cont = 0;
    dosis_actual = 1;
    }
t_20s = 31;
while(t_20s);
ajuste_perfusor(dosis_actual,5000,&fallo);
t_20s = 3;
while(t_20s);
i++;
if (i==90){ /* scroll horizontal */
    i=45;
    borra_grafico();
    menu_4_v("DOSIS","MENU","","");
    display_string_v(0,18,"              ");
```

```
display_string_v(0,19,"          ");

dibuja_ejes();

dosis[0]=dosis[45];

presion[0]=presion[45];

for (k=1;k<=44;k++){

    dosis[k]=dosis[k+45];

    presion[k]=presion[k+45];

    dibuja_presion_dosis(k,presion[k],dosis[k-1],dosis[k]);

    }

}

display_string_h (0,16,"mmHg");

display_string_h (1,17,"med");

display_char (2,19,presion_media);

display_string_h (3,17,"max");

display_char (4,19,presion_maxima);

display_string_h (5,17,"min");

display_char (6,19,presion_minima);

display_string_h (8,17,"INF");

display_var (9,18,dosis_actual);

switch (1_pressed){

        case 'A':  forma=forma+2;

                   display_string_h (0,16,"    ");

                   display_string_h (1,17,"   ");

                   display_string_h (2,17,"   ");

                   display_string_h (3,17,"   ");

                   display_string_h (4,17,"   ");

                   display_string_h (5,17,"   ");

                   display_string_h (6,17,"   ");

                   dosis_actual=input_var(9,18,dosis_inicial);
```

```
                     if (!modo)

                        {

                           dosis_auxiliar = dosis_actual/10;

                   valor_inicial.infusion_inicial = dosis_auxiliar;

                           inicia_control(valor_inicial);

                        }

                     break; /* Opcion sup. izda. */

              case 'B':  fin_bloque=1;

                     break; /* Opcion inf. izda. */

              default :  break;

                 }

       l_pressed=0;

       dosis_ant = dosis_actual;

          }

    }

    finaliza_conversion();

    ajuste_perfusor(0,0,&fallo);

}

void control_automatico(void)

{

    control_de_presion(0);

}


void control_manual(void)


    control_de_presion(1);

}
```

```
void monitorizar_presion(void)
{
    extern char l_pressed;
    extern unsigned char punto[1664], grafico_sim[110];
    char fin_monitorizacion=0;
    unsigned char i,j,k,num_punto,t=16,simulado,
                  b,x_byte,y_byte,minimo=180,
                  presion_media,presion_maxima=0,presion_minima=0xff;
    unsigned short total;
    unsigned long p_sum;


    borra_grafico();


    menu_4_grande("","",
                  "SIMULADA","",
                  "","",
                  "  REAL","");
    display_string_h(1,0,"                        ");
    display_string_h(2,0,"                        ");
    display_string_h(3,0,"    MONITORIZACION    ");
    display_string_h(4,0,"                        ");
    j=1;
    while(j){
      l_pressed=0;
      while (!l_pressed);
      switch (l_pressed){
            case 'B': simulado=1;
                      j=0;
                      break;
```

EP 0 408 483 B1

```
                    case 'D': simulado=0;
                              j=0;
                              break;

                    default : break;
                    }
          }
     borra_grafico();
     menu_4_v("STOP ","MENU","Per.+","Per.-");
     inicializa_conversion();
     l_pressed=0;
     while(!fin_monitorizacion){
        y_byte=180;
        total=0;
        while((y_byte>(minimo+2))&&(total<2000)){
                    *adctl_addr=0;  /* SCAN=0 MULT=1    AN0 */
                    while(!fin_conversion());
                    y_byte=(*adr1_addr);
                    y_byte=y_byte/4;
                    y_byte=71-y_byte;
                    total++;
                    }


        minimo=180;
        p_sum=0;
        total=0;
        *adctl_addr=0;


        num_punto=0;
        if (simulado) t=16;
```

```
for (i=2;i<=14;i++) {
  for (b=0;b<=7;b++) {
    for (k=0;k<=t-1;k++) {
      x_byte=i;
      y_byte=*adr1_addr;
      *adct1_addr=0;
      if(!k) presion_minima = presion_maxima = y_byte;
      if(y_byte<presion_minima) presion_minima=y_byte;
      if(y_byte>presion_maxima) presion_maxima=y_byte;
      p_sum+=y_byte;
      y_byte=y_byte/4;
      y_byte=71-y_byte;
      if ((y_byte>80)) y_byte=80;
      if (simulado) y_byte= grafico_sim [num_punto];
      if (y_byte<minimo) minimo=y_byte;
      punto[(short)(num_punto+(110*k))]=y_byte;
            /* Almacena valor actual */
      if (i<14)
*yx_display=(short)(i+1+20*punto[(short)(8+num_punto+(110*k))]);
                                        e    l    s    e
  *yx_display=(short)(2+20*punto[(short)(b+(110*k))]);
        *portb_addr=32 ¦ *portb_addr;
        *vis_addr=0x0a;
        *portb_addr=128 ¦ *portb_addr;
        *portb_addr=127 & *portb_addr;
        *portb_addr=223 & *portb_addr;
        *vis_addr=*x_display;
        *portb_addr=128 ¦ *portb_addr;
        *portb_addr=127 & *portb_addr;
```

24

```
*portb_addr=32 | *portb_addr;
*vis_addr=0x0b;
*portb_addr= 128 | *portb_addr;
*portb_addr=127 & *portb_addr;
*portb_addr=223 & *portb_addr;
*vis_addr=*y_display;
*portb_addr=128 | *portb_addr;
*portb_addr=127 & *portb_addr;
*portb_addr=32 | *portb_addr;
*vis_addr=0x0e;
*portb_addr=128 | *portb_addr;
*portb_addr=127 & *portb_addr;
*portb_addr=223 & *portb_addr;
*vis_addr=b;
*portb_addr=128 | *portb_addr;
*portb_addr=127 & *portb_addr;


*yx_display=(short)(x_byte+20*y_byte)

*portb_addr=32 | *portb_addr;
*vis_addr=0x0a;
*portb_addr=128 | *portb_addr;
*portb_addr=127 & *portb_addr;
*portb_addr=223 & *portb_addr;
*vis_addr=*x_display;
*portb_addr=128 | *portb_addr;
*portb_addr=127 & *portb_addr;
*portb_addr=32 | *portb_addr;
*vis_addr=0x0b;
```

25

```
        *portb_addr= 128 ¦ *portb_addr;

        *portb_addr=127 & *portb_addr;

        *portb_addr=223 & *portb_addr;

        *vis_addr=*y_display;

        *portb_addr=128 ¦ *portb_addr;

        *portb_addr=127 & *portb_addr;

        *portb_addr=32 ¦ *portb_addr;

        *vis_addr=0x0f;

        *portb_addr=128 ¦ *portb_addr;

        *portb_addr=127 & *portb_addr;

        *portb_addr=223 & *portb_addr;

        *vis_addr=b;

        *portb_addr=128 ¦ *portb_addr;

        *portb_addr=127 & *portb_addr;

        total++;

        }

    num_punto++;

    }

}

num_punto--;

presion_media=0;

while (p_sum>total){p_sum=p_sum-total;presion_media++;}

display_string_h (0,15,"mmHg");

display_string_h (2,15,"med");

display_char (3,17,presion_media);

display_string_h (5,15,"max");

display_char (6,17,presion_maxima);

display_string_h (8,15,"min");

display_char (9,17,presion_minima);
```

26

```
    switch (1_pressed){
        case 'A':  menu_4_v("SIGUE","       "," ","       ");
                   1_pressed=0;
                   while (1_pressed!='A');
                   menu_4_v("STOP","MENU","Per.+","Per.-");
                   break; /* Opcion sup. izda. */
        case 'B':  fin_monitorizacion=1;
                   break; /* Opcion inf. izda. */
        case 'C':  if (t<16) {
                            t=t*2;
                            borra_grafico();
                            menu_4_v("STOP","MENU","Per.+","Per.-")

                   }
                   break; /* Opcion sup. dcha. */
        case 'D':  if (t>1)  {
                            t=t/2;
                            borra_grafico();
                            menu_4_v("STOP","MENU","Per.+","Per.-");

                   }

                   break; /* Opcion inf. dcha. */
        default :  break;
        }
    1_pressed=0;
    }
finaliza_conversion(); /*  Apaga el conversor AD */
```

27

```
    }


void finalizar_programa(void)
{
    extern char l_pressed;


    borra_grafico();


    menu_4_grande("","",
                  "  SI","",
                  "","",
                  "  NO","");
    display_string_h(1,0,"      F I N       ");
    display_string_h(2,0,"        del        ");
    display_string_h(3,0,"    Control de      ");
    display_string_h(4,0,"  Presion Arterial ?");



    while(1){
       l_pressed=0;
       while (!l_pressed);
       switch (l_pressed){
               case 'B':  fin_programa=1;
                          borra_grafico();
                          display_string_h(2,1,"Programa terminado");
                          break;
               case 'D':  fin_programa=0;
                          break;
```

```
                    default :   break;
                    }

            }

    }


void opciones_varias(void)

    {

        extern char l_pressed,kb_pressed;

        char fin_bucle;

        fin_bucle = 0;

        while(!fin_bucle){

            borra_grafico();        /* Vacia la pantalla */


            menu_4_grande("  LUZ    ","PANTALLA",

                          "  FIN    ","PROGRAMA",

                          " MENU    ","PRINCIPAL",

                          " SALIDA ","ARCHIVOS");

        l_pressed=0;

        while (!l_pressed);     /* Mientas no se elija una opcion */

        switch (l_pressed){

                case 'A':   borra_grafico();

                            display_string_h(3,2,"0 ILUMINAR FONDO");

                            display_string_h(5,2,"1 APAGAR  FONDO");

                            kb_pressed = 0;

                            while(!kb_pressed);

                            switch (kb_pressed) {

                                case '0': reset_bit (1,portb_addr);

                                          break;

                                case '1': set_bit (1,portb_addr);
```

```
                                        break;
                        default : break;
                        }
                break;
        case 'B':  finalizar_programa();
                break;
        case 'C':  fin_bucle = 1;
                break;
        case 'D':  break;
        default :  break;
        }
    }
}
main()
{
    extern char l_pressed;
    char fin_programa=0;
    *init_addr = 0x01;
    *pioc_addr = 0x43;
    *ddrc_addr = 0xff;
    inicializa_irq();
    intercept_serial_6811();
    inicializa_serie_6811();
    inicializa_serie_68681();
    inicializa_newtimer();
    modo_grafico();
    borra_grafico();


    menu_1();
```

```
        t_20s = 100;

        while(t_20s);


        while(!fin_programa){

          borra_grafico();

          menu_4_grande(" Control","AUTOMAT.",
                        " Control"," MANUAL ",
                        " MONITOR","        ",
                        "OPCIONES"," VARIAS ");


          l_pressed=0;

          while (!l_pressed);

          switch (l_pressed){
                    case 'A':  control_automatico();
                               break; /* Opcion sup. izda. */
                    case 'B':  control_manual();
                               break; /* Opcion inf. izda. */
                    case 'C':  monitorizar_presion();
                               break; /* Opcion sup. dcha. */
                    case 'D':  opciones_varias();
                               break; /* Opcion inf. dcha. */
                    default :  break;
                    }
            }


        while(1);



          }
```

**Claims**

1. An apparatus for delivering vasoactive drugs by means of an infusion pump, commanded by a computerized device which automatically controls and adjusts the dosage in a closed-loop in accordance to the evolution of monitorized arterial blood pressure values of the patient, and is used to

31

maintain mean arterial blood pressure within a predefined range of values, comprising: a controller, that includes a micro-controller, ROM memory, RAM memory, three RS-232 serial communication outputs, real time clock, visual and acoustic indicators, two parallel input/output communication ports of eight bit each, eight analogical/digital conversion channels, graphic display means, keyboard means, power supply means with battery support means and battery charger means; an arterial blood pressure monitor means; an infusion pump means for intravenously injecting the drug into the patient; an infusion rate computation means wherein a predetermined target gap for the Mean Arterial Pressure (MAP) is provided, characterised in that: said infusion rate computation means uses a fuzzy logic based control algorithm whereby:

a).- a transient dose is infused when MAP is outside the target gap,

b).- the transient dose is computed through a non-linear relationship with actual MAP, the proportionality coefficient increasing exponentially with MAP,

c).- an optimal dose is continuously updated and increased or decreased more slowly following a logarithmic incrementation coefficient, this logarithmic relationship being adapted with the patient's varying situation by means of a growth factor,

d).- the optimal dose is infused when MAP is within the target zone.

## Patentansprüche

1. Ein Gerät zum Dosieren von gefässaktiven Drogen durch eine Infusionspumpe mit computerisierter Steuerung, die im geschlossenen Wirkungsweg in Abhängigkeit von den Veränderungen des arteriellen Blutdrucks des Patienten die Dosierung steuert und regelt. Das Gerat dient dazu, den mittleren Blutdruck des Patienten konstant zwischen zwei zu definierenden Grenzwerten zu halten. Es besteht aus einer Steuereinheit mit Mikroprozessor, einem ROM- und einem RAM-Speicher, drei seriellen Schnittstellen vom Typ RS 232, einer Echtzeituhr, optischen und akustischen Anzeigegeräten, zwei parallelen Schnittstellen mit je 8 bits, 8 Kanälen zur Analog-Digital-Umwandlung, Mechanismen zur graphischen Darstellung, Tastaturen, Netzteilen, Mechanismen zur Monitorisierung des arteriellen Blutdrucks (MAP), einer Infusionspumpeneinheit zur intravenösen Injektion der Droge, sowie aus Recheneinheiten zur Bestimmung der Infusionsgeschwindigkei und zur Berechnung des objektiven Toleranzbereichs der Mittelwerte des arteriellen Blutdrucks (MAP).

Arbeitsweise: die Recheneinheit zur Bestimmung der Infusionsgeschwindigkeit operiert mit einem Steueralgorithmus, der ermittelt wird durch eine diffuse Logik zur Erfüllung der folgenden Aufgaben:

a) Sobald der Mittelwert des arteriellen Blutdrucks (MAP) den objektiven Toleranzbereich verlässt, wird eine übergangsdosis infundiert.

b) Diese übergangsdosis wird berechnet aufgrund eines nichtlinearen Verhältnisses zu den aktuellen Mittelwerten des arteriellen Blutdrucks und zu einem Proportionalitätskoeffizienten, der sich exponentiell mit dem Mittelwert des arteriellen Blutdrucks vergrössert.

c) Daneben findet die permanente Aktualisierung einer Optimaldosis statt, die in Abhängigkeit von einem logarithmischen Wachstumskoeffizienten langsam erhöht oder reduziert wird, wobei das logarithmische Verhältnis mithilfe eines Wachstumsfaktors den Zustandsänderungen des Patienten angepasst wird.

d) Solange sich der Mittelwert des arteriellen Blutdrucks innerhalb des objektiven Toleranzbereichs befindet, wird die Optimaldosis infundiert.

## Revendications

1. Un appareil pour doser les drogues vasoactives à l'aide d'une pompe à infusion, contrôlée par un dispositif computérisé qui contrôle et régle automatiquement le dosage en boucle fermée, conformément à l'évolution des valeurs de pression sanguine artérielle du patient, et qui est utilisé pour maintenir la pression sanguine artérielle moyenne dans une bande de valeurs prédéfinie. Il se compose de: un contrôleur qui inclut un microcontrôleur, une mémoire ROM, une mémoire RAM, trois sorties de communication en série RS232, une horloge de temps réel, des indicateurs visuels et acoustiques, deux ports de communication d'entrée/sortie en parallèle de huit bits chacun, huit canaux de conversation analogique/digitale, des mécanismes de présentation graphique, des mécanismes de clavier, des mécanismes de source d'alimentation, des mécanismes de motorisation du la pression sanguine artérielle, (MAP), des mécanismes de pompe d'infusion afin d'injecter le drogue par voie intraveinause au patient, des mécanismes de calcul de la vitesse d'infusion fournissant une bande objective de valeurs de pression moyenne artérielle (MAP), caractérisé par, le mécanisme de calcul de la vitesse d'infusion emploie un algorythme de contrôle taxé en logique diffuse au moyen de laquelle:

a) une dose transitoire est infusée lorsque la pression artérielle moyenne (MAP) se situe en dehors de la bande objective.

b) La dose transitoire est calculée au moyen d'un rapport non linéaire entre les valeurs actuelles de pression artérielle moyenne et un coefficient de proportionnalité qui croit exponentiellement avec la pression artérielle moyenne.

c) Vue dose parfaite est continuellement mise à jour et augmente ou décroit plus lentement selon un coefficient d'augmentation logarythmique, ce rapport logarythmique étant adapté selon le changement de situation du patient par un facteur de croissance.

d) La dose parfaite est infusée lorsque la pression artérielle moyenne se trouve dans la bande objective.

SINGLE FIGURE